# FASCICULE DE BREVET EUROPEEN

(11) **EP 1 653 917 B1**
(45) Date de publication et mention de la délivrance du brevet: **07.05.2008**
(21) Numéro de dépôt: 04703412.9
(22) Date de dépôt: 20.01.2004
(51) Int. Cl.: A61K 8/18

(54) **EMULSIONS HUILE-DANS-EAU CONCENTREES ET DILUEES, STABLES**
STABILE, ÖL-IN-WASSER, KONZENTRIERTE UND DÜNNE EMULSIONEN
CONCENTRATED AND DILUTED STABLE OIL/WATER EMULSIONS

(30) Priorité: 12.08.2003 FR 0309861
(43) Date de publication de la demande: 10.05.2006
(73) Titulaire: J & C INTERNATIONAL, 92635 Gennevilliers (FR)
(72) Inventeur: ROSSOW, Véronique, F-33120 Arcachon (FR); ROSSOW, Nicolas, F-95550 Bessancourt (FR); ROSSOW, Jean, F-92300 Levallois-Perret (FR)
(74) Mandataire: Touati, Catherine
(86) Numéro de dépôt international: PCT/FR2004/000122
(87) Numéro de publication internationale: WO 2005/025533

(56) Documents cités:
- WO-A-02/06403
- FR-A- 2 041 400
- FR-A- 2 738 830
- FR-A- 2 825 099

## Description

L'invention concerne des émulsions huile-dans-eau qui présentent une stabilité remarquable et sont ou bien concentrées ou bien diluées ainsi que leurs procédés de préparation. Elle concerne également un procédé simple de formulation mettant en oeuvre ces émulsions.

Dans de nombreux domaines techniques, les émulsions sont très utilisées étant donné qu'elles permettent l'association durable d'au moins deux phases liquide-liquide non miscibles.

Les émulsions peuvent être classées en deux grandes catégories, les émulsions huile-dans-eau et les émulsions eau-dans-huile. Dans les émulsions huile-dans-eau la phase continue est l'eau, dans cette phase sont dispersées des gouttelettes d'huile. Inversement, dans les émulsions eau-dans-huile, la phase continue est l'huile dans laquelle sont dispersées des gouttelettes d'eau.

Dans la présente demande, on entend par « émulsion stable », une émulsion qui ne présente pas de phénomènes de coalescence ou de séparation de phases après conservation pendant 6 mois à la température ambiante, de préférence après conservation pendant 12 mois à la température ambiante, et plus préférentiellement encore pendant 18 mois à la température ambiante.

Suivant le domaine technique dans lequel les émulsions sont mises en oeuvre, les qualités requises sont différentes. Cependant, certaines qualités sont souhaitées pour toutes les émulsions, notamment la stabilité au cours du temps et aux variations de températures, mais encore des qualités spécifiques en terme notamment de rhéologie, de stabilité vis à vis de variations de pH, de stabilité aux électrolytes.

En cosmétique, en pharmacie ou en alimentaire par exemple, les émulsions doivent présenter de nombreuses qualités supplémentaires, notamment en termes d'aspect, de compatibilité avec des substances actives, de sensation au toucher, de sensation en bouche, de parfum, d'acceptation dermatologique ou alimentaire, etc.

Les émulsions obtenues par les méthodes conventionnelles présentent cependant l'inconvénient d'être très sensibles, notamment à la température et à la dilution ou à des paramètres physico-chimiques tels que la présence d'électrolytes ou les variations de pH.

Toute modification, aussi minime soit elle, de la composition d'une émulsion, peut avoir une influence sur sa stabilité et ceci de façon difficilement prédictible. Ainsi, une modification entraîne la plupart du temps une modification des qualités de l'émulsion, voire un déphasage de l'émulsion et nécessite donc la mise au point d'un nouveau procédé de formulation approprié.

Dès lors, de façon classique, lorsque l'on souhaite introduire un nouveau produit dans une émulsion afin de lui conférer une propriété donnée ou un aspect donné, ou bien augmenter ou diminuer la teneur en l'un des constituants, il est nécessaire de définir par tâtonnements successifs un nouveau protocole complet de formulation.

Les présents inventeurs ont recherché un moyen de simplifier le travail des formulateurs.

Après des recherches longues et approfondies, ils ont trouvé un procédé extrêmement simple et extrêmement pratique de formulation d'émulsions complexes huile-dans-eau, qui nécessite un faible apport énergétique et conduit à des émulsions présentant une stabilité remarquable.

Ce procédé a été décrit dans la demande de brevet FR 0309861 au nom de la Société Demanderesse et comprend les étapes selon lesquelles :
- on détermine Les propriétés et fonctionnalités de l'émulsion huile-dans-eau finale souhaitée ;
- on choisit des émulsions élémentaires présentant chacune au moins l'une des propriétés ou fonctionnalités devant être présentées par l'émulsion finale;
- éventuellement on dilue au moins l'une des émulsions élémentaires avec une phase aqueuse;
- on mélange les différentes émulsions élémentaires, dont éventuellement certaines ont préalablement été diluées ;
- on dilue éventuellement avec une phase aqueuse, les étapes de dilution et de mélange étant réalisées de préférence sans exercer de forces de cisaillement ; chacune desdites émulsions élémentaires étant une émulsion huile-dans-eau stable, contenant
   - au moins un corps gras simple ou complexe choisi dans le groupe comprenant notamment les esters d'acides gras, les cires, les beurres, les cire-esters, les huiles naturelles, synthétiques, ou minérales, les huiles hydrogénées et leurs mélanges ;
   - au moins un tensio-actif, de préférence un tensio-actif non ionique de préférence un tensio-actif non-ionique, plus particulièrement choisi dans le groupe comprenant les esters d'acides gras de polyglycérol éventuellement éthoxylés, les éthoxylats d'alcools;
   - au moins un co-tensioactif, choisi parmi les composés hydrophiles, de préférence comprenant au moins un groupe hydroxyle, choisis notamment dans la famille des polyols,
   - de l'eau,
   - et éventuellement au moins un agent actif,
ce par quoi l'émulsion complexe résultante est stable.

Dans cette demande de brevet, il est indiqué que chacune des émulsions élémentaires peut être préparée par le procédé dit « Detergent-phase », tel qu'il est décrit notamment dans les articles suivants Fragrance Journal 1993-4, 35-40, H. Sagitani, J.Dispersion Sci. 9, 115 (1988) et H. Sagitani, Y. Hirai, K. Nabet, Chem.Soc.Jpn, 35, 102 (1986).

Les inventeurs ont maintenant trouvé que ce procédé est tout particulièrement avantageux et efficace lorsque chacune des émulsions élémentaires est une émulsion huile-dans-eau stable, obtenue à partir d'une pré-émulsion air-dans-eau comprenant:
- un tensio-actif de préférence un tensio-actif non-ionique, plus particulièrement choisi dans le groupe comprenant les esters d'acides gras de polyglycérol éventuellement éthoxylés, les éthoxylats d'alcools;
- un co-tensioactif choisi parmi les composés hydrophiles, de préférence comprenant au moins un groupe hydroxyle, choisis notamment dans la famille des polyols,
- une phase aqueuse,
dans des rapports choisis dans la zone de structure liquide-cristal ordonnée du diagramme de phases de ces trois éléments,et/ou dans des rapports choisis de façon à ce que la structure du mélange de ces trois constituants observée au microscope optique en lumière polarisée présente des caractéristiques de biréfringence,
un corps gras simple ou complexe étant ajouté à ladite pré-émulsion par mélange modéré, de préférence sans exercer de force de cisaillement, ledit corps gras étant choisi dans le groupe comprenant notamment les esters d'acides gras, les cires, les beurres, les cire-esters, les huiles naturelles, synthétiques dont notamment les silicones et leurs dérivés, ou minérales, les huiles hydrogénées et leurs mélanges .

L'émulsion est une émulsion huile-dans-eau se présentant sous la forme d'un gel, elle est concentrée, très stable et peut être diluée.

Au sens de l'invention, une structure présente des caractéristiques de biréfringence dès lors qu'une observation en lumière polarisée de cette structure est possible, c'est-à-dire dès lors qu'il y a formation d'une image. A contrario, l'observation en lumière polarisée d'une structure ne présentant pas de caractéristiques de biréfringence sera impossible, il n'y aura pas de formation d'image, on observera un fond noir.

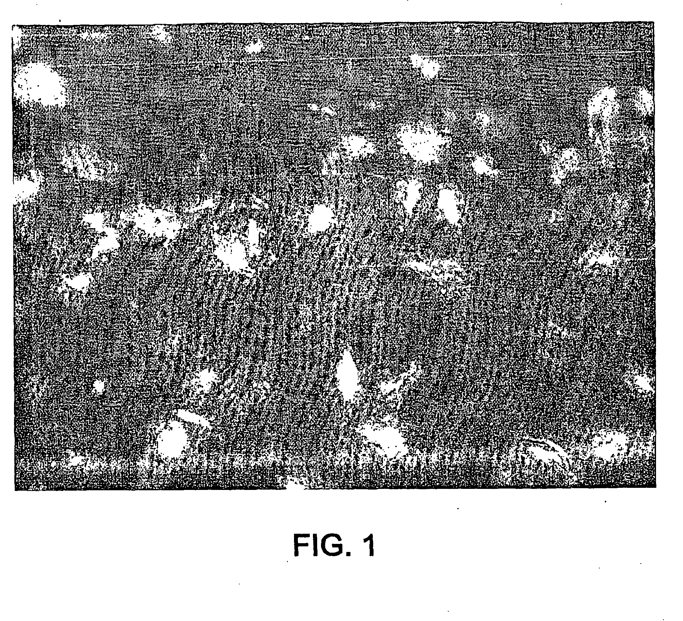

La structure du mélange tensio-actif, co-tensioactif et phase aqueuse dans les proportions requises, telle qu'observée au microscope en lumière polarisée est illustrée sur la figure 1.

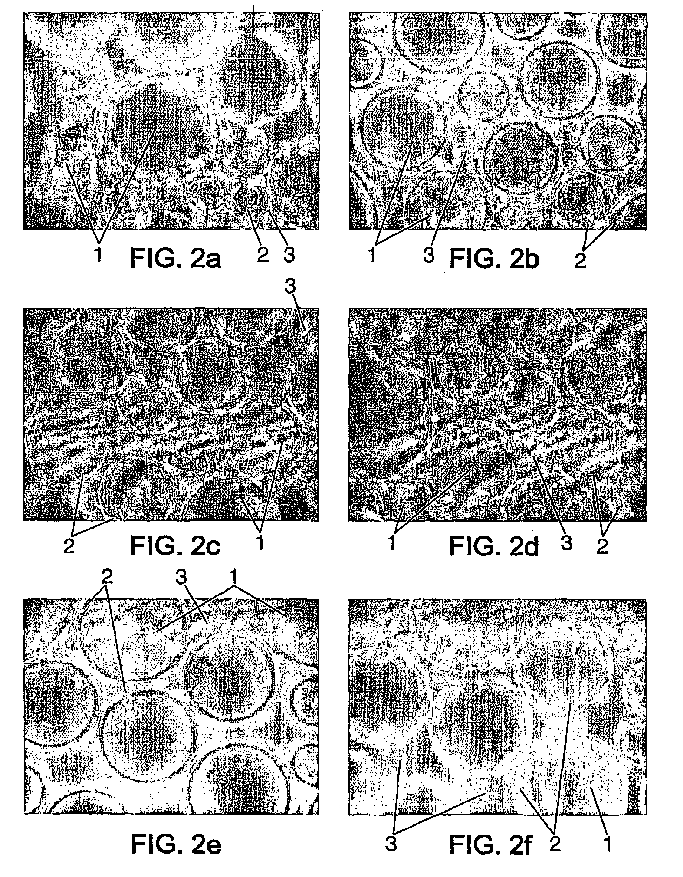

La pré-émulsion air-dans-eau à partir de laquelle est préparée l'émulsion huile-dans-eau stable conforme à l'invention est une pâte blanche. Les globules d'air constituant cette émulsion air-dans-eau sont visibles au microscope optique en lumière polarisée, leurs surfaces présentant des caractéristiques de biréfringence, comme ceci apparaît sur la figure 2

Sans vouloir être liée par aucune théorie, la Société Demanderesse pense que la structure observée sur cette figure est une structure ordonnée biréfringente lamellaire.

Dans la présente description, le terme « huile » utilisé de façon générale désigne les huiles d'origine naturelle, végétale, animale, d'origine marine ou d'origine synthétique dont notamment les silicones et leurs dérivés" les huiles minérales telles que les huiles de paraffine, les huiles hydrogénées mais également les autres corps gras simples ou complexes tels que notamment les esters d'acides gras, les cires, les cire-esters, les beurres, et leurs mélanges.

La phase aqueuse peut être de l'eau, de l'eau déionisée, de l'eau stérilisée, ou bien, une émulsion huile-dans-eau, ou bien une solution aqueuse dans laquelle sont dilués, dispersés ou suspendus, des agents actifs. Le pH de la phase aqueuse peut varier dans une large gamme de pH=2 environ à pH=10 environ.

Selon un mode de réalisation particulièrement avantageux de l'invention, le tensio-actif est choisi dans le groupe des tensioactifs dérivant du glycérol, comprenant les dérivés de lécithines, les esters d'acides gras de polyglycérol éventuellement éthoxylés, de préférence des esters d'acides gras de décaglycérol de HLB supérieure ou égale à 13, de façon davantage préférée encore le laurate, le myristate, le stéarate, l'isostéarate, ou l'oléate de décaglycérol, et leurs mélanges.

Les esters d'acides gras de polyglycérol sont particulièrement appropriés dans des émulsions destinées à venir en contact avec la peau ou les muqueuses car ils sont très peu irritants.

Les tensio actifs à base de dérivés de lécithine sont quant à eux très utiles pour les émulsions alimentaires.

Le co-tensioactif utilisé est quant à lui choisi parmi les composés hydrosolubles, de préférence les composés comportant au moins une fonction hydroxyle, notamment les polyols ou leurs mélanges, de préférence le diglycérol.

La proportion d'eau, de tensio-actif et de co-tensioactif est choisie dans la zone de structure liquide-cristal ordonnée du diagramme de phase de ces trois éléments et/ou de façon à ce que la structure du mélange de ces trois constituants telle qu'observée au microscope optique en lumière polarisée présente des caractéristiques de biréfringence.

L'observation au microscope en lumière polarisée d'un mélange de ces trois constituants dans des proportions se trouvant en dehors de la zone d'intérêt ne permet de distinguer aucune structure, il n'y a pas de biréfringence.

L'émulsion air-dans-eau comprend, pour 100 parties en poids d'eau, de 300 à 50, de préférence de 200 à 120 parties de tensio-actif et de 300 à 50 , de préférence de 180 à 100 parties de co-tensioactif.

L'émulsion air-dans-eau peut bien entendu-inclure des additifs classiquement utilisés dans les émulsions, par exemple des stabilisants, des modificateurs de pH, des anti-oxydants.

L'émulsion huile-dans-eau sous forme de gel, peut en outre contenir au moins un agent actif hydrosoluble ou liposoluble. Bien entendu dans le cas d'un agent actif hydrosoluble, celui-ci sera présent dans la partie aqueuse de l'émulsion et dans le cas d'un agent actif liposoluble, celui-ci sera présent dans la phase huileuse.

L'invention concerne également un procédé de préparation d'une émulsion huile-dans-eau stable comprenant successivement .
a. le choix d'un tensio-actif de préférence d'un tensio-actif non-ionique, plus particulièrement choisi dans le groupe comprenant les esters d'acides gras de polyglycérol éventuellement éthoxylés, les éthoxylats d'alcools et d'un co-tensioactif choisi parmi les composés hydrophiles, de préférence comprenant au moins un groupe hydroxyle, choisis notamment dans la famille des polyols;
b. le mélange modéré, de préférence sans exercer de force de cisaillement, du tensio-actif, du co-tensioactif et d'une phase aqueuse dans des proportions données par la zone cristal-liquide ordonnée du diagramme de phase de ces constituants et/ou dans des proportions choisies de façon à ce que la structure du mélange de ces trois constituants observée au microscope optique en lumière polarisée présente des caractéristiques de biréfringence, le mélange étant réalisé jusqu'à l'obtention d'une pré-émulsion air-dans-eau blanche visible par observation au microscope en lumière polarisée, la surface des globules d'air présentant des caractéristiques de biréfringence, comme ceci apparaît sur la figure 2;
c. l'incorporation dans cette pré-émulsion air-dans-eau d'un corps gras simple ou complexe par mélange modéré, de préférence sans exercer de force de cisaillement,jusqu'à l'obtention d'un gel, et
d. éventuellement l'addition d'une phase huileuse,
e. éventuellement l'addition d'une phase aqueuse, de façon à obtenir une émulsion blanche.

Le corps gras simple ou complexe est ajouté par fractions successives sous agitation lente sans exercer de force de cisaillement.

L'étape b) de formation de l'émulsion air-dans-eau est suivie par observation au microscope optique en lumière polarisée. Quand les constituants tensio-actif, co-tensioactif et phase aqueuse ou eau sont grossièrement mélangés dans les proportions souhaitées, on observe une structure présentant des caractéristiques de biréfringence telle qu'illustrée sur la Figure 1 qui est une photographie de l'image observée au microscope optique sous lumière polarisée du mélange grossier des 3 constituants. La présence de cette structure présentant des caractéristiques de biréfringence est la preuve que les proportions entre les 3 constituants sont satisfaites. Le mélange est ensuite poursuivi jusqu'à ce que l'observation au microscope de l'émulsion réalisée soit telle qu'illustrée sur les figures 2a à 2f, c'est-à-dire que l'on observe en lumière polarisée des bulles d'air 1 dont les surfaces 2 présentent des caractéristiques de biréfringence, et qui sont entourées d'une phase aqueuse 3.

Les différentes figures 2 (2a à 2f) représentent la même émulsion air-dans-eau photographiée selon des champs différents (figures 2a à 2e), la figure 2f correspondant à la figure 2e mais avec une profondeur de champ différente.

L'obtention de l'émulsion blanche finale est réalisée par la dilution du gel avec une phase aqueuse simple ou complexe, sous agitation lente et préférentiellement sans cisaillement.

La phase aqueuse utilisée à l'étape e) peut être différente de celle utilisée dans l'étape b) de formation de l'émulsion air-dans-eau.

Au sens de la présente invention, aussi bien l'émulsion huile-dans-eau se présentant sous forme de gel, obtenue à l'issue de l'étape c) que l'émulsion blanche obtenue après addition d'une phase aqueuse à l'issue de l'étape e) sont dites « émulsions concentrées ».

Compte tenu de la stabilité remarquable des émulsions obtenues par le procédé décrit ci-dessus, il est possible = de les diluer.

Ainsi, selon un autre mode de réalisation, le procédé selon l'invention comprend une étape ultérieure f) de dilution à l'aide de façon générale d'une phase aqueuse contenant éventuellement une substance active hydrophile par mélange de ladite phase aqueuse à l'émulsion huile-dans-eau stable préalablement obtenue.

Selon encore un autre mode de réalisation, le procédé de l'invention peut comprendre à la place de l'étape f) de dilution, avant ou après celle-ci, une étape g) d'addition par mélange modéré, de préférence sans cisaillement d'une autre émulsion huile-dans-eau stable selon l'invention ou d'une autre émulsion huile-dans-eau classique.

Il est ainsi possible de mélanger différentes émulsions huile-dans-eau stables de l'invention entre elles afin d'obtenir une émulsion huile-dans-eau complexe. L'émulsion huile-dans-eau complexe est elle aussi stable.

L'opération mécanique de mélange des différents composés constitutifs de la pré-émulsion air-dans-eau, de l'émulsion huile-dans-eau, de dilution et de mélange de diverses émulsions élémentaires se fait de façon modérée, sans qu'il soit nécessaire d'exercer un cisaillement, celui-ci étant même préférentiellement évité.

Cette opération est réalisée, par exemple, à l'aide d'une ancre ou d'un planétaire.

Chaque étape de mélange ou de dilution peut se faire à une température inférieure à 70°C, de préférence inférieure à 40°C et plus préférentiellement encore à température ambiante.

La quantité de phase aqueuse ajoutée au gel à l'étape e) est fonction du type d'émulsion concentrée et de son usage. Cette quantité représente environ 1/5 de la formule pour une émulsion destinée à être : (a) pulvérisable et à forte teneur en phase huileuse interne, ou bien (b) lait et à forte teneur en phase huileuse interne, 1/4 de la formule pour une émulsion destinée à être « excipient » crème ou lait à teneur modérée en phase huileuse , et 1/2 de la formule pour une émulsion destinée à être pulvérisable et à teneur en phase huileuse modérée à faible.

Les émulsions huile-dans-eau obtenues à n'importe quel stade du procédé, présentent une stabilité remarquable et des caractéristiques physiques communes.

Selon le type de tensio-actif(s) mis en oeuvre dans les émulsions huile-dans-eau stables de l'invention, la taille des particules ou gouttelettes d'huile varie, cependant la répartition granulométrique est homogène au sein d'une émulsion huile-dans-eau.

Selon un mode de réalisation avantageux, l'émulsion huile-dans-eau est fine, c'est-à-dire que le diamètre moyen des particules ou gouttelettes d'huile est inférieur à 10µm environ, généralement il est inférieur à 1µm environ, de préférence il est compris entre 150 et 750nm environ.

Les émulsions huile-dans-eau stables de l'invention comprennent de 0,05% à 95% en poids d'huile, de préférence de 30 à 92% en poids d'huile.

De façon avantageuse, la teneur en tensioactif dans l'émulsion conforme à l'invention est au plus d'environ 20% en poids, de préférence d'au plus environ 10% en poids et plus préférentiellement encore d'au plus environ 5% en poids

De façon particulièrement avantageuse, les émulsions conformes à l'invention sont des émulsions cosmétiques, pharmaceutiques ou alimentaires qui comprennent respectivement au moins un agent ayant une activité cosmétique, pharmaceutique ou alimentaire.

Selon le choix de la phase huileuse, du tensioactif et/ou de l'agent actif incorporé dans la phase aqueuse et/ou dans la phase huileuse, l'émulsion présentera une ou des propriétés ou fonctionnalités particulières caractéristiques.

Ainsi, certaines émulsions peuvent avoir des propriétés physico-chimiques caractéristiques, par exemple une rhéologie donnée, une texture particulière, une aptitude à l'étalement, et/ou des propriétés sensorielles, notamment un parfum, une teinte ou couleur.

Ces propriétés sont conférées par exemple par la présence dans l'émulsion d'au moins un agent modifiant les caractéristiques physico-chimiques et/ou sensorielles de l'émulsion, qui est choisi dans le groupe comprenant notamment les colorants, agents de rhéologie, agents de texture, agents de charge, parfum ou leurs mélanges.

D'autres émulsions pourront être dotées, par exemple, d'une fonctionnalité gustative, d'une fonctionnalité ou activité pharmaceutique, dermatologique, cosmétique, etc.

Une fonctionnalité gustative ou utile pour des émulsions alimentaires est par exemple une saveur particulière, une aromatisation, une amélioration de la sensation en bouche, un enrichissement en vitamines ou en acides gras essentiels.

Cette fonctionnalité est conférée par un arôme naturel ou de synthèse, un agent sucrant, un agent salant, des édulcorants, des vitamines, des sels minéraux, des acides gras essentiels, un agent d'amertume, un agent rafraîchissant, etc.

Une fonctionnalité ou activité pharmaceutique peut être conférée par la présence d'une huile pharmaceutique active ou bien d'au moins une substance pharmaceutiquement active, ou bien d'un agent permettant une modification de la libération de la substance active.

Une fonctionnalité ou activité cosmétique ou dermatologique peut être une activité de lutte contre le vieillissement, de cicatrisation, de limitation de la sécrétion de séborrhée, de nettoyage, de protection, contre les UV, d'hydratation, d'émollience, d'astringence, etc.

Ces fonctionnalités sont apportées par une phase huileuse ayant des propriétés cosmétiques et/ou par l'ajout dans la phase huileuse ou aqueuse d'agents actifs, tels qu'un agent anti-rides, un filtre UV, un agent hydratant, un agent tenseur, un émollient, un agent astringent, un agent moussant, un agent de toucher, un agent rafraîchissant, des vitamines, huiles essentielles, protéines, acides aminés, acides de fruits, etc.

Bien entendu, une émulsion peut comprendre plusieurs propriétés et/ou fonctionnalités.

La quantité d'agents actifs présents dans l'émulsion conforme à l'invention dépend bien entendu de leur nature. La quantité d'agents actifs hydrosolubles, qui sont présents dans la phase aqueuse, peut varier dans une très large plage de valeurs pouvant aller jusqu'à environ 80% en poids du poids total de l'émulsion, plus généralement elle est d'au plus environ 10%, et de préférence de l'ordre de 5% environ. La, quantité d'agents actifs liposolubles, présents dans la phase huileuse, peut être jusqu'à 92% environ en poids, de préférence de 30 à 50% en poids et plus préférentiellement encore de 1 à 10% en poids du poids total de l'émulsion.

Parmi les émulsions huile-dans-eau conformes à l'invention, on peut distinguer des émulsions « excipients » et des émulsions « actives ».

Les émulsions « excipients » jouent le rôle des excipients en formulation classique, elles confèrent le corps ou la base de la formule. Leur rôle n'est pas d'apporter une fonctionnalité ou propriété spéciale à la formule finale. Elles sont utilisées soit seules, soit comme corps de formulation. Ces formulations sont destinées à être diluées ou bien à l'aide d'une phase aqueuse lorsqu'elles sont utilisées seules ou bien avec des émulsions actives lorsqu'elles sont utilisées comme corps de formulation. Dans les deux cas, afin d'éviter tout phénomène de crémage, et garder une viscosité suffisante, elles peuvent être diluées jusqu'à un seuil maximal qui dépend notamment du système tensio-actif/co-tensioactif mis en oeuvre et qui est d'au moins 50% de l'émulsion de base dans le cas des « bases » pour émulsions pulvérisables, et d'au moins 40% de l'émulsion de base dans le cas de « bases » pour crèmes ou pour lait. Ces pourcentages peuvent cependant varier, notamment selon le point de fusion des phases huileuses utilisées pour les bases pour crèmes ou pour laits.

Les émulsions « actives » sont quant à elles formulées pour pouvoir être additionnées au corps de formule en la plus petite quantité possible, tout en apportant la plus grande efficacité possible (c'est-à-dire que l'actif soit présent en quantité la plus grande possible).

Une difficulté supplémentaire pour les formulateurs consiste en la préparation de formules pulvérisables, c'est-à-dire présentant un viscosité telle qu'elles peuvent être pompées aisément et projetées en fines gouttelettes.

Les présents inventeurs ont trouvé que les émulsions stables conformes à l'invention pouvaient se présenter sous la forme d'émulsions pulvérisables. Les émulsions pour produits pulvérisables conformes à l'invention peuvent être préparées de différentes façons, à partir d'émulsions concentrées. Ces émulsions concentrées sont de type crème, c'est-à-dire ne s'écoulant pas librement, de type lait, c'est-à-dire s'écoulant librement mais non directement pulvérisable ou bien de type pulvérisable.

Selon l'émulsion concentrée de départ, le toucher de l'émulsion pulvérisable finale sera différent de même que sa concentration finale en phase huileuse.

Les émulsions concentrées de type crème, sont riches en huile, et contiennent de 45 à 90% en poids du poids total de l'émulsion concentrée d'huile, de préférence environ 68%, elles présentent un rapport tensioactif/co-tensioactif compris entre 0,5 et 1,5, de préférence d'environ 1,22 et un rapport tensioactif/huile compris entre 0,01 et 0,15, de préférence d'environ 0,05. Ces émulsions concentrées riches en huile peuvent être diluées jusqu'à une teneur minimale en huile dans l'émulsion diluée pulvérisable comprise entre 35% et 70%, de préférence d'environ 40,0 % en poids.

Les émulsions concentrées de type lait, peuvent présenter, une teneur en huile modérée, et contiennent alors de 10 à 70% d'huile en poids du poids total de l'émulsion concentrée, de préférence environ 30% dans le cas ou elles sont formulées à base de corps gras solides à température ambiante, ou bien elles présentent une teneur en huile élevée, de préférence 68% dans le cas ou elles sont préparées à l'aide de corps gras et de tensio-actifs liquides à température ambiante, elles présentent généralement un rapport tensioactif/co-tensioactif compris entre 0,5 et 2,5, de préférence d'environ 0,9 à 1,3 et un rapport tensioactif/huile compris généralement entre 0,01 et 1. Ces émulsions concentrées ayant une teneur en huile de préférence de 68% peuvent être pulvérisables lorsqu'elles sont diluées, et ce jusqu'à une teneur minimale en huile dans l'émulsion diluée pulvérisable comprise entre 1 et 60%, de préférence d'environ 42% en poids.

Les émulsions concentrées élémentaires pulvérisables présentant une faible teneur en huile sont particulièrement adaptées à la préparation de concentrés « actifs » (qui tels que formulés sont pulvérisables, même sans être dilués) et sont destinés à être ajoutés en petites quantités à des émulsions « excipients ». Elles contiennent de 1 à 40% d'huile en poids du poids total de l'émulsion concentrée, de préférence environ 30%, elles présentent un rapport tensioactif/co-tensioactif compris entre 1,5 et 10, de préférence d'environ 1,05 et un rapport tensioactif/huile compris entre 0,1 et 3, de préférence d'environ 0,33. Ces émulsions concentrées à faible teneur en huile peuvent être diluées seules sans phénomènes de crémage, jusqu'à une teneur minimale en huile dans l'émulsion diluée comprise entre 0,05 et 10%, de préférence d'environ 5% en poids.

Les émulsions élémentaires destinées à être diluées par une phase huileuse se présentent sous la forme de gels et sont obtenues à l'issue de l'étape c) du procédé conforme à l'invention ont une teneur en huile comprise entre 10 et 70% du poids total, et préférentiellement de 60%, un rapport tensioactif/cotensioactif de 0,01 à 10, et préférentiellement de 0,97 à 1,22 et un rapport tensioactif/huile de 0,01 à 0,5 et préférentiellement de 0,17 à 0,27. La quantité de phase huileuse pouvant être ajoutée est de 0,1 à 100%, il n'y a pas de valeur préférentielle, car cette dilution s'effectue selon ce que le formulateur souhaite ajouter.

Compte tenu du fait que les émulsions huile-dans-eau conformes à l'invention sont très stables et peuvent être mélangées entre elles et que l'émulsion résultante est elle-même très stable et possède l'ensemble des propriétés, fonctionnalités et/ou caractéristiques de chacune des émulsions qui la constituent avec éventuellement des synergies particulières lorsque deux émulsions « actives » sont mélangées, synergies pouvant apporter de nouvelles propriétés, de façon pratique, il est donc possible de formuler de façon très simple des émulsions complexes présentant l'ensemble souhaité de fonctionnalités, caractéristiques et propriétés.

Ces émulsions sont dites complexes car dans la phase aqueuse, qui peut elle-même comprendre des agents actifs, sont dispersés de façon stable plusieurs types de particules ou gouttelettes d'huile.

L'invention concerne donc également un procédé de formulation d'une émulsion huile-dans-eau complexe comprenant les étapes selon lesquelles :
- on détermine les propriétés et fonctionnalités de l'émulsion huile-dans-eau finale souhaitée ;
- on choisit des émulsions élémentaires présentant chacune au moins l'une des propriétés ou fonctionnalités devant être présentées par l'émulsion finale;
- éventuellement on dilue au moins l'une des émulsions élémentaires avec une phase aqueuse;
- éventuellement on dilue au moins l'une des épuisions élémentaires spécifiques avec une phase huileuse,
- on mélange les différentes émulsions élémentaires, dont éventuellement certaines ont préalablement été diluées ;
- on dilue éventuellement avec une phase aqueuse
- on dilue éventuellement avec une phase huileuse;
les étapes de dilution et de mélange étant réalisées de préférence sans exercer de forces de cisaillement ;
chacune desdites émulsions élémentaires étant une émulsion huile-dans-eau stable selon l'invention.

L'opération mécanique de mélange des différentes émulsions élémentaires se fait de façon modérée, sans qu'il soit nécessaire d'exercer un cisaillement, celui-ci étant même préférentiellement évité.

Cette opération est réalisée, par exemple, à l'aide d'une ancre ou d'un planétaire.

Chaque étape de mélange ou de dilution peut se faire à une température inférieure à 70°C, de préférence inférieure à 40°C et plus préférentiellement encore à la température ambiante.

Le nombre d'émulsions élémentaires pouvant être combinées entre elles n'est pas un facteur limitant.

Cependant, de façon générale, le procédé de formulation conforme à l'invention comprend le mélange d'au plus 20 émulsions élémentaires, de préférence d'au plus 10 émulsions élémentaires, et de façon davantage préférée encore de 2 à 8 émulsions élémentaires.

Compte tenu du fait que chaque propriété ou fonctionnalité est apportée par une émulsion élémentaire différente, et que les différentes émulsions élémentaires sont combinées entre elles, il est possible de préparer conformément à l'invention des émulsions complexes comprenant simultanément des agents actifs incompatibles. Il s'agit principalement d'agents actifs lipophiles.

L'incompatibilité de certaines matières actives entre elles n'est donc pas un obstacle à la formulation d'émulsions conformément au procédé de l'invention.

Compte tenu de cette simplicité de formulation, il est possible de mettre en place des bornes ou automates de formulation sur mesure qui permettent au consommateur de personnaliser des formulations existantes ou bien de créer des formulations spécifiques.

Ainsi, un autre aspect de l'invention consiste en la mise en place d'automates permettant la fabrication d'émulsions, en particulier d'émulsions cosmétiques, à partir d'instructions saisies par un utilisateur sur un écran ou tout autre dispositif de dialogue, et portant sur les qualités, propriétés ou caractéristiques de l'émulsion désirée. L'automate peut en outre être programmé pour délivrer l'émulsion accompagnée de sa composition en différents constituants par ordre décroissant de concentrations, afin de répondre aux exigences légales.

La présente invention porte également sur les émulsions huile-dans-eau complexes ainsi obtenues qui présentent une stabilité remarquable et peuvent comprendre un mélange de matières actives incompatibles.

Elle porte également sur un procédé de préparation de produits contenant au moins une émulsion huile-dans-eau complexe.

Ce procédé comporte les étapes consistant en la préparation d'au moins une émulsion huile-dans-eau complexe selon l'invention, le mélange de cette (ou ces) émulsion(s) avec les autres constituants du produit.

Lorsque plusieurs émulsions huile-dans-eau complexes selon l'invention sont utilisées, elles sont avantageusement préalablement mélangées entre elles avant l'addition des autres constituants.

L'invention va être décrite plus en détail dans les exemples suivants qui sont donnés uniquement à titre d'illustration de l'invention et qui ne sont pas limitatifs.

### EXEMPLES

Dans les exemples qui suivent, les émulsions huile-dans-eau ont été préparées avec une étape préalable de formation d'une émulsion air-dans-eau.

Le protocole de préparation de l'émulsion air-dans-eau est.le suivant .

On mélange sans cisaillement, à l'aide d'une ancre ou d'un planétaire, de l'eau distillée,le tensio-actif et le co-tensioactif

Dès que ces 3 composants sont mis ensemble et homogénéisés grossièrement, on observe au microscope optique en lumière polarisée une structure présentant des caractéristiques de biréfringence telle qu'illustrée par la Figure 1. Le mélange est poursuivi jusqu'à l'obtention d'une pâte blanche épaisse dont l'apparence par observation au microscope en lumière polarisée est illustrée par la Figure 2, sur laquelle apparaissent des bulles d'air. 1 dont les surfaces 2 présentent des caractéristiques de biréfringence, les interstices 3 entre les bulles d'air étant remplis de phase aqueuse.

Dans tous les exemples, l'observation au microscope est réalisée en lumière polarisée avec un microscope Olympus CX 41, grossissement ×1000.

### Exemple 1 : préparation d'une émulsion « base crème »

On prépare une émulsion huile-dans-eau présentant la composition centésimale donnée dans le Tableau 1 ci-après, de la façon suivante.

**Tableau 1**

| Ingrédients | | | % en poids |
|---|---|---|---|
| Nom commercial | Producteur | Appellation INCI | |
| Diglycérine S | Sakamoto | Diglycerin | 3,92 |
| S-Face M-1001 | Sakamoto | Polyglyceryl-10 Myristate | 4,8 |
| Parleam | NOF | Hydrogenated Polyisobutene | 46,4 |
| Purester 40 | Strahl & Pitsch | Stearyl Behenate | 8 |
| Huile de Coprah | Cognis | Hydrogenated Palm Kernel Oil | 8 |
| Esters de Jojoba | A&E Connock | Jojoba Esters | 2,4 |
| Lanette 16 | Cognis | Cetyl Alcohol | 3,2 |
| Eau distillée | | Aqua | 23,08 |
| Methylparaben | | Methylparaben | 0,2 |
| | | | |
| Total | | | 100 |

### a/ Préparation de l'émulsion air-dans-eau

A une température légèrement supérieure à la température de fusion de la phase huileuse, on mélange sans cisaillement, à l'aide d'une ancre ou d'un planétaire, 32,8 g d'eau distillée, 39,2 g de diglycérine et 48g de Polyglyceryl-10 Myristate.

### b/ Préparation de l'émulsion huile-dans-eau :

On introduit alors, sous agitation modérée sans cisaillement et à environ 55°C, toutes les 2,5 minutes environ, par fractions de 10g quatre fois de suite, puis par fractions de 20 g quatre fois de suite, puis par fractions de 40 g jusqu'à complète incorporation, la phase huileuse qui comprend un mélange de 464g de polyisobutène hydrogéné, 80 g de Stearyl Behenate, 80 g d'huile de coprah hydrogénée, 24g d'esters de Jojoba et 32g d'alcool cétylique.

Quand la quantité totale de phase huileuse est ajoutée, le mélange est arrêté et on obtient un gel.

A ce gel on ajoute, toujours par mélange modéré sans cisaillement, 198 g d'eau distillée et chauffée à environ 55°C ,et 2.0 g de méthylparaben.

On obtient 1 kg d'émulsion blanche stable ne s'écoulant pas librement après refroidissement à température ambiante, et diluable avec une phase aqueuse et/ou avec une émulsion huile-dans-eau.

### Exemple 2 Préparation d'une émulsion « base lait »

On prépare une émulsion huile-dans-eau présentant la composition centésimale donnée dans le Tableau 2 ci-après, de la façon suivante.

**Tableau 2**

| Ingrédients | | | % en poids |
|---|---|---|---|
| Nom commercial | Producteur | Appellation INCI | |
| Diglycérine S | Sakamoto | Diglycerin | 7,84 |
| S-Face S-1001 | Sakamoto | Polyglyceryl-10 Stéarate | 9,6 |
| Parleam | NOF | Hydrogenated Polyisobutene | 46,96 |
| Purester 24 | Strahl & Pitsch | Lauryl Laurate | 9,04 |
| Eau distillée | | Aqua | 26,36 |
| Methylparaben | | Methylparaben | 0,2 |
| Total | | | 100 |

### a/ Préparation de l'émulsion air-dans-eau :

A environ 50°C, on mélange sans cisaillement, à l'aide d'une ancre ou d'un planétaire, 65,6 g d'eau distillée, 78,4 g de diglycérine et 96 g de Polyglyceryl-10 Stéarate.

### b/ Préparation de l'émulsion huile-dans-eau :

On introduit alors, sous agitation modérée sans cisaillement et à environ 50°C, toutes les 2,5 minutes environ, par fractions de 10 g quatre fois de suite, puis par fractions de 20 g quatre fois de suite, puis par fractions de 30 g quatre fois de suite, puis par fractions de 40 g jusqu'à complète incorporation, la phase huileuse qui comprend un mélange de 469,6g de polyisobutène hydrogéné et 90,4 g de Lauryl laurate.

Quand la quantité totale de phase huileuse est ajoutée, le mélange est arrêté et on obtient un gel.

A ce gel on ajoute, toujours par mélange modéré sans cisaillement, 65,6 g d'eau distillée préalablement chauffée à environ 50° C et 2 g de méthylparaben.

On obtient 1 kg d'émulsion blanche stable s'écoulant librement, n'étant pas pulvérisable, diluable avec une phase aqueuse et/ou avec une émulsion huile-dans-eau.

### Exemple 3 : Préparation d'une émulsion concentrée pour

### « émulsion pulvérisable riche en huile »

On prépare une émulsion huile-dans-eau présentant la composition centésimale donnée dans le Tableau 3 ci-après, de la façon suivante.

**Tableau 3**

| Ingrédients | | | % en poids |
|---|---|---|---|
| Nom commercial | Producteur | Appellation INCI | |
| Diglycérine S | Sakamoto | Diglycerin | 3,92 |
| S Face IS-1001 | Sakamoto | Polyglyceryl-10 Isostearate | 4,8 |
| Huile d'amande douce | Bertin | Sweet almond oil | 68 |
| Eau distillée | | Aqua | 23,08 |
| Methylparaben | | Methylparaben | 0,2 |
| Total | | | 100 |

### a/ Préparation de l'émulsion air-dans-eau :

A la température ambiante, on mélange sans cisaillement, à l'aide d'une ancre ou d'un planétaire, 32,8 g d'eau distillée, 39,2g de diglycérine et 48g de Polyglyceryl-10 Isostéarate.

### b/ préparation de l'émulsion huile-dans-eau :

On introduit alors, sous agitation modérée sans cisaillement et à la température ambiante, toutes les 2,5 minutes environ, par fractions de 10g quatre fois de suite, puis par fractions de 20 g quatre fois de suite, suite, puis par fractions de 30 g quatre fois de suite, puis par fractions de 40 g jusqu'à complète incorporation, la phase huileuse qui est constituée de 680 g d'huile d'amande douce.

Quand la quantité totale de phase huileuse est ajoutée, le mélange est arrêté et on obtient un gel transparent.

A ce gel on ajoute, toujours par mélange modéré sans cisaillement, 198 g d'eau distillée et 2 g de méthylparaben.

On obtient 1 kg d'émulsion blanche stable s'écoulant librement, non pulvérisable, et diluable avec une phase aqueuse et/ou avec une émulsion huile-dans-eau.

### Exemple 4 Préparation d'une émulsion concentrée pour « émulsion sprayable à teneur en huile modérée à faible »

On prépare une émulsion huile-dans-eau présentant la composition centésimale donnée dans le Tableau 4 ci-après, de la façon suivante.

**Tableau 4**

| Ingrédients | | | % en poids |
|---|---|---|---|
| Nom commercial | Producteur | Appellation INCI | |
| Diglycérine S | Sakamoto | Diglycerin | 9,5 |
| S-Face M-1001 | Sakamoto | Polyglyceryl-10 Myristate | 10 |
| Huile d'amande douce | Bertin | Sweet almond oil | 30 |
| Eau distillée | | Aqua | 50,3 |
| Methylparaben | | Methylparaben | 0,2 |
| Total | | | 100 |

### a/ Préparation de l'émulsion air-dans-eau :

A la température ambiante, on mélange sans cisaillement, à l'aide d'une ancre ou d'un planétaire, 5g d'eau distillée, 95g de diglycérine et 100g de Polyglyceryl-10 Myristate.

### b/ Préparation de l'émulsion air-dans-eau :

On introduit alors, sous agitation modérée sans cisaillement et à la température ambiante, toutes les 2,5 minutes, par fraction de 10g quatre fois de suite, puis par fractions de 20 g quatre fois de suite, puis par fractions de 30 g quatre fois de suite, puis par fractions de 40 g jusqu'à complète incorporation la phase huileuse qui est constituée de 300 g d'huile d'amande douce. Quand la quantité totale de phase huileuse est ajoutée, le mélange est arrêté et on obtient un gel.

A ce gel on ajoute, toujours par mélange modéré sans cisaillement, 498 g d'eau distillée et 2 g de méthylparaben.

On obtient 1 kg d'émulsion blanche stable s'écoulant librement, pouvant être pulvérisée, et diluable avec une phase aqueuse et/ou avec une émulsion huile-dans-eau.

### Exemple 5 : Préparation d'une émulsion concentrée pour « émulsion pulvérisable à teneur en phase huileuse modérée à faible »

On prépare une émulsion huile-dans-eau présentant la composition centésimale donnée dans le Tableau 5 ci-après, de la façon suivante.

**Tableau 5**

| Ingrédients | | | % en poids |
|---|---|---|---|
| Nom commercial | Producteur | Appellation INCI | |
| Glycérine | Dow | Glycerin | 9,5 |
| S Face L 1001 | Sakamoto | Polyglyceryl-10 Laurate | 10 |
| Parleam | NOF | Hydrogenated Polyisobutene | 30 |
| Eau distillée | | Aqua | 50,3 |
| Methylparaben | | Methylparaben | 0,2 |
| Total | | | 100 |

### a/ Préparation de l'émulsion air-dans-eau :

A la température ambiante, on mélange sans cisaillement, à l'aide d'une ancre ou d'un planétaire, 5 g d'eau distillée, 95 g de glycérine et 100g de Polyglyceryl-10 laurate.

### b/ Préparation de l'émulsion huile-dans-air :

On introduit alors, sous agitation modérée sans cisaillement et à la température ambiante, les 300g de polyisobutène hydrogéné par fractions de 10 g quatre fois de suite puis par fraction de 20 g 4 fois de suite, puis par fraction de 30g pour la quantité restante.

On obtient un gel.

A ce gel on ajoute, toujours par mélange modéré sans cisaillement, et par fraction de 30 à 60 ml les 503 g d'eau distillée et 2 g de méthylparaben.

On obtient 1 kg d'émulsion blanche stable s'écoulant librement, pulvérisable, et diluable avec une phase aqueuse et/ou avec une émulsion huile-dans-eau.

### Exemple 6 Préparation d'une émulsion concentrée et diluable « solaire »

On prépare une émulsion huile-dans-eau présentant la composition centésimale donnée dans le Tableau 6 ci-après, de la façon suivante.

**Tableau 6**

| Ingrédients | | | % en poids |
|---|---|---|---|
| Nom commercial | Producteur | Appellation INCI | |
| Diglycérine S | Sakamoto | Diglycerin | 3,92 |
| S-Face M-1001 | Sakamoto | Polyglyceryl-10 Myristate | 4,8 |
| Huile de pépins de Canneberge | Aromtech | Cranberry Seed Oil | 30 |
| Parsol MCX | Roche Vitamins | Ethyl hexyl methoxycinnamate | 30 |
| Purester 24 | Strahl & Pitsch | Lauryl Laurate | 8 |
| Eau distillée | | Aqua | 23,08 |
| Methylparaben | | Methylparaben | 0,2 |
| Total | | | 100 |

### a/ Préparation de l'émulsion air-dans-eau :

A la température ambiante, on mélange sans cisaillement, à l'aide d'une ancre ou d'un planétaire, 32,8 g d'eau distillée, 39,2g de diglycérine et 48g de Polyglyceryl-10 Myristate.

### b/ préparation de l'émulsion huile-dans-eau :

On introduit alors, sous agitation modérée sans cisaillement et à la température ambiante, toutes les 2,5 minutes environ, par fraction de 10g quatre fois de suite, puis par fractions de 20 g quatre fois de suite, puis par fractions de 30 g quatre fois de suite, puis par fractions de 40 g jusqu'à complète incorporation, la phase huileuse qui comprend un mélange de 300g d'huile de pépins de canneberge, 300g d'ethyl hexyl methoxycinnamate et 80g de Lauryl laurate.

Quand la quantité totale de phase huileuse est ajoutée, le mélange est arrêté et on obtient un gel.

A ce gel on ajoute, toujours par mélange modéré sans cisaillement, 198 g d'eau distillée et 2 g de méthylparaben.

On obtient 1 kg d'émulsion blanche stable s'écoulant librement, non pulvérisable, et diluable avec une phase aqueuse et/ou avec une émulsion huile-dans-eau.

### Exemple 7 : Préparation d'une émulsion concentrée diluable par une phase huileuse

On prépare une émulsion huile-dans-eau présentant la composition centésimale donnée dans le Tableau 7 ci-après, de la façon suivante.

**Tableau 7**

| Ingrédients | | | % en poids |
|---|---|---|---|
| Nom commercial | Producteur | Appellation INCI | |
| Diglycérine S | Sakamoto | diglycerin | 9,8 |
| S-Face IS-1001 | Sakamoto | Polyglyceryl-10 Laurate | 6,0 |
| S-Face M-1001 | Sakamoto | Polyglyceryl-10 Myristate | 6,0 |
| Parleam | NOF | Hydrogenated Polyisobuten | 58,7 |
| Purester 24 | Strahl & Pitsch | Lauryl Laurate | 11,3 |
| Eau distillée | | Aqua | 8,0 |
| Methylparaben | | Methylparaben | 0,2 |
| Total | | | 100 |

### a/ Préparation de l'émulsion air-dans-eau :

A la température ambiante, on mélange sans cisaillement, à l'aide d'une ancre ou d'un planétaire, 80 g d'eau distillée, 98 g de Diglycérine, 60 g de Polyglyceryl-10 Myristate et 60 g de Polyglyceryl-10 Isostearate.

### b/ Préparation de l'émulsion huile-dans-air

On introduit alors, sous agitation modérée sans cisaillement et à la température ambiante, toutes les 2,5 minutes, par fractions de 10g quatre fois de suite puis par fractions de 20 g quatre fois de suite, puis par fractions de 30 g quatre fois de suite, puis par fractions de 40g le reste des 700 g de la phase huileuse qui comprend un mélange de 587 g de Polyisobutène hydrogéné et 113 g de Lauryl Laurate.

Quand la quantité totale de phase huileuse est ajoutée, le mélange est arrêté et on obtient un gel transparent.

On obtient 1 kg de gel transparent stable et diluable avec une phase huileuse puis une phase aqueuse, ou bien directement avec une phase aqueuse.

### Exemple 8 : Préparation d'une émulsion diluable « anti-ride »

On prépare une émulsion huile-dans-eau présentant la composition centésimale donnée dans le Tableau 8 ci-après, de la façon suivante.

**Tableau 8**

| Ingrédients | | | % en poids |
|---|---|---|---|
| Nom commercial | Producteur | Appellation INCI | |
| glycérine | Dow | glycerin | 9,5 |
| S-Face M-1001 | Sakamoto | Polyglyceryl-10 Myristate | 10 |
| Huile de pépins de myrtilles | Aromtech | Bilberry Seed Oil | 30 |
| Purester 24 | Strahl & Pitsch | Lauryl Laurate | 3 |
| Eau distillée | | Aqua | 47,3 |
| Methylparaben | | Methylparaben | 0,2 |
| Total | | | 100 |

### a/ Préparation de l'émulsion air dans-eau :

A la température ambiante, on mélange sans cisaillement, à l'aide d'une ancre ou d'un planétaire, 5 g d'eau distillée, 95g de glycérine et 100g de Polyglyceryl-10 Myristate.

### b/préparation de l'émulsion huile-dans-eau :

On introduit alors, sous agitation modérée sans cisaillement et à la température ambiante, toutes les 2,5 minutes environ, par fractions de 10g quatre fois de suite, puis par fractions de 20 g quatre fois de suite, puis par fractions de 30 g quatre fois de suite, puis par fractions de 40 g jusqu'à complète incorporation, la phase huileuse qui comprend un mélange de 300g d'huile de pépins de myrtilles, et 30g de Lauryl laurate.

Quand la quantité totale de phase huileuse est ajoutée, le mélange est arrêté et on obtient un gel.

A ce gel on ajoute, ,toujours par mélange modéré sans cisaillement, 498g d'eau distillée et 2g de méthylparaben.

On obtient 1kg d'émulsion blanche stable qui s'écoule librement, pouvant être pulvérisée, et pouvant être diluée.

### Exemple 9 : Préparation d'une émulsion diluable « anti-inflammatoire »

On prépare une émulsion huile-dans-eau présentant la composition centésimale donnée dans le Tableau 9 ci-après, de la façon suivante.

**Tableau 9**

| Ingrédients | | | % en poids |
|---|---|---|---|
| Nom commercial | Producteur | Appellation INCI | |
| glycérine | Dow | glycerin | 9,5 |
| S-Face M-1001 | Sakamoto | Polyglyceryl-10 Myristate | 10 |
| Huile de pépins de cassis | Aromtech | Blackcurrant Seed Oil | 30 |
| Purester 24 | Strahl & Pitsch | Lauryl Laurate | 3 |
| Eau distillée | | Aqua | 47,3 |
| Methylparaben | | Methylparaben | 0,2 |
| Total | | | 100 |

L' émulsion est préparée de la même façon que pour l'exemple 8 en remplaçant dans les mêmes quantités l'huile de pépins de myrtilles par de l'huile de pépins de cassis.

On obtient une émulsion stable qui s'écoule librement et qui peut être pulvérisée.

### Exemple 10 : Préparation d'une émulsion diluable « régénération cellulaire »

On prépare une émulsion huile-dans-eau présentant la composition centésimale donnée dans le Tableau 10 ci-après, de la façon suivante.

**Tableau 10**

| Ingrédients | | | % en poids |
|---|---|---|---|
| Nom commercial | Producteur | Appellation INCI | |
| glycérine | Dow | glycerin | 9,5 |
| S-Face M-1001 | Sakamoto | Polyglyceryl-10 Myristate | 10 |
| Extrait de Tourbe | Aromtech | Peat extract | 3 |
| Huile d'amande douce | Bertin | Sweet almond oil | 27 |
| Purester 24 | Strahl & Pitsch | Lauryl Laurate | 3 |
| Eau distillée | | Aqua | 47,3 |
| Methylparaben | | Methylparaben | 0,2 |
| Total | | | 100 |

L'émulsion est préparée de la même façon que pour l'exemple 9 en remplaçant l'huile de pépins de myrtilles par 270 g d'huile d'amande douce et 30 g d' extrait de Tourbe. On obtient une émulsion blanche stable, qui s'écoule librement et qui peut être pulvérisée.

### Exemple 11 : Dilution des émulsions de base

Chacune des émulsions de base préparées dans les exemples 1 à 5, a été diluée avec de l'eau. Dans le tableau ci-dessous est indiquée la quantité maximale d'eau qu'il a été possible d'ajouter pour que les émulsions restent « stables », c'est à dire qu'il n'y ait pas apparition du phénomène de crémage. Dans tous les cas, quelque soit le degré de dilution, aucune de ces émulsions ne présentera de phénomènes de séparation de phases.

| Emulsions de base | Quantité maximale d'eau pouvant être ajoutée (en g pour 100g de concentré) | % minimal d' huile dans émulsions diluées |
|---|---|---|
| Base crème (exemple 1) | 94 | 35 |
| Base lait (exemple 2) | 40 | 40 |
| Base pulvérisable riche en huile (exemple 3) | 36 | 50 |
| Base pulvérisable contenant environ 30% d'huile (exemple 4) | 100 | 15 |
| Base pulvérisable contenant 30% d'huile (ex.5) | 400 | 6 |

Cas particuliers : Exemple 7 : émulsions diluables avec une phase huileuse puis dilutions avec une phase aqueuse

| Phase huileuse pouvant être incorporée | Quantité maximale de phase huileuse pouvant être ajoutée (en g pour 100g de de l'exemple 7 concentré) | Dilution maximale par une phase aqueuse de l'exemple 7 dilué avec une phase huileuse |
|---|---|---|
| Mélange 70 : 30 de cyclométhicone (DC 345 de Dow Corning) et Lauryl Laurate (Purester 24 de Strahl & Pitsch) | 100 | 50% |
| Vitamin A Palmitate 1.7 m.i.U./g (Roche) | 20 | 40% |

### Exemple 12 : mélanges de différentes émulsions

On a réalisé diverses émulsions complexes en mélangeant les émulsions fonctionnelles des exemples 6 et. 8 à 10 avec les émulsions de base des exemples 1 à 5, et 7.

Dans le tableau ci-dessous sont indiquées les quantités minimales d'émulsions actives pouvant être mélangées à des émulsions de base pour que la fonctionnalité de l'émulsion active soit manifestée par l'émulsion résultante.(Ces valeurs inférieurs ne sont pas limitatives et dépendront de la composition de l'émulsion active).

Sont également indiquées les quantités maximales pouvant être ajoutées. Ces quantités maximales sont définies par des considérations législatives.

| **Emulsions actives** | **% minimum** | **%maximum** |
|---|---|---|
| Solaire (ex.6) | 1 | 25 |
| Anti-ride (ex.8) | 1 | 100 |
| Anti-inflammatoire (ex.9) | 1 | 100 |
| Régénération cellulaire (ex. 10) | 1 | 100 |

### Exemple 13 : Formulation d'une émulsion corporelle hydratante et pulvérisable :

On choisit les émulsions élémentaires suivantes :
- (B) émollience et base émulsion fluide
- (G) silicone
- (D) parfum agrumes

Ces émulsions élémentaires sont introduites dans les proportions données dans le tableau 11 ci-dessous, avec une phase aqueuse contenant de l'eau et 0,2% de méthylparaben en tant que conservateur, dans un flacon.

On mélange alors de façon modérée jusqu'à l'obtention d'une émulsion homogène. On obtient ainsi une émulsion fluide pouvant être appliquée sur la peau par pulvérisation, qui est de couleur blanche et qui est stable.

**Tableau 11**

| **Émulsion élémentaire** | **% en poids** |
|---|---|
| A | 20,8 |
| B | 50,0 |
| C | 2,0 |
| Phase aqueuse | 27,2 |

La composition de chaque émulsion élémentaire est donnée ci-après .
A) émollience et base émulsion fluide

| Phases | Ingrédients | | | % en poids |
|---|---|---|---|---|
| | Nom commercial | Producteur | Appellation INCI | |
| A- | Eau distillée | - | Aqua | 3,28 |
| | Diglycérine S | Sakamoto | Diglycerin | 3,92 |
| | M-1001 | Sakamoto | Polyglycerol-10 Myristate | 4,8 |
| B- | Parleam | NOF | Hydrogenated Polyisobutene | 68,0 |
| C- | Eau distillée | - | Aqua | 18,8 |
| | Aristoflex AVC | Clariant | Amonium Acryloyldimethyltaurate /VP copolymer | 0,2 . |
| | Methylparaben | Tri-K | Methylparaben | 1,0 |

(B) Silicone :

| Phases | Ingrédients | | | % en poids |
|---|---|---|---|---|
| | Nom commercial | Producteur | Appellation INCI | |
| A- | Eau distillée | - | Aqua | 3,28 |
| | Diglycérine S | Sakamoto | Diglycerin | 3,92 |
| | IS-1001 | Sakamoto | Polyglyceryl-10 Isostearate | 2,4 |
| | M-1001 | Sakamoto | Polyglycerol-10 Myristate | 2,4 |
| B- | DC 345 | Dow Corning | Cyclomethicone | 48,0 |
| | Purester 24 | Strahl & Pitsch | Lauryl Laurate | 20,0 |
| C- | Eau distillée | - | Aqua | 19,8 |
| | Methylparaben | Tri-K | Methylparaben | 0,2 |

(C) parfum agrumes

| Phases | Ingrédients | | | % en poids |
|---|---|---|---|---|
| | Nom commercial | Producteur | Appellation INCI | |
| A- | Eau distillée | | Aqua | 3,28 |
| | Diglycérine S | Sakamoto | Diglycerin Polyglyceryl-10 | 3,92 |
| | S-Face M-1001 | Sakamoto | Myristate | 4,8 |
| B- | Parfum Citron | Fragrance Oils | Fragrance | 16,0 |
| | Parleam 4 | NOF | Hydrogenated Polyisobutene | 4,0 |
| | Purester 24 | Strahl & Pitsch | Lauryl Laurate | 12,0 |
| | Parleam | NOF | Hydrogenated Polyisobutene | 36,0 |
| C- | Eau distillée Méthylparaben | Tri-K | Aqua Methylparaben | 19,8 0,2 |

Chacune des émulsions élémentaires (A) à (C) est préparée de la façon suivante :

A la température ambiante, on mélange les ingrédients de la phase A jusqu'à l'obtention d'une émulsion « air-dans-eau » selon le protocole ci-dessus.

Puis, sous agitation lente on ajoute peu à peu la phase B, et enfin on ajoute la phase C. On obtient ainsi une émulsion huile-dans-eau, fluide et très blanche.

La répartition granulométrique de l'émulsion résultante est monodisperse, et ne présente aucune coalescence dans le temps.

## Revendications

1. Emulsion huile-dans-eau stable, obtenue à partir d'une pré-émulsion air-dans-eau comprenant:
- un tensio-actif de préférence un tensio-actif non-ionique, plus particulièrement choisi dans le groupe comprenant les esters d'acides gras de polyglycérol éventuellement éthoxylés, les éthoxylats d'alcools;
- un co-tensioactif choisi parmi les composés hydrophiles, de préférence comprenant au moins un groupe hydroxyle, choisis notamment dans la famille des polyols,
- une phase aqueuse,
dans des rapports choisis dans la zone de structure liquide-cristal ordonnée du diagramme de phases de ces trois éléments, et/ou dans des rapports choisis de façon à ce que la structure du mélange de ces trois constituants observée au microscope optique en lumière polarisée présente des caractéristiques de biréfringence,
un corps gras simple ou complexe étant ajouté à ladite pré-émulsion par mélange modéré, de préférence sans exercer de force de cisaillement, ledit corps gras étant choisi dans le groupe comprenant notamment les esters d'acides gras, les cires, les beurres, les cire-esters, les huiles naturelles, synthétiques, ou minérales, les huiles hydrogénées et leurs mélanges .

2. Emulsion selon la revendication 1, **caractérisée par le fait que** la pré-émulsion air-dans-eau est une pâte blanche dont les globules d'air constitutifs peuvent être observés en lumière polarisée, leurs surfaces présentant des caractéristiques de biréfringence.

3. Emulsion selon la revendication 1 ou la revendication 2, **caractérisée par le fait que** le tensio-actif est choisi dans le groupe des dérivés de glycérol comprenant les dérivés de lécithine, les esters d'acides gras de polyglycérol éventuellement éthoxylés, de préférence des esters d'acides gras de décaglycérol de HLB supérieure ou égale à 13, de façon davantage préférée encore le laurate, le myristate, le stéarate, l'isostéarate ou l'oléate de décaglycérol, et leurs mélanges et **par le fait que** le co-tensioactif est un polyol, de préférence le diglycérol.

4. Emulsion selon l'une quelconque des revendications 1 à 3, **caractérisée par le fait que** la pré-émulsion air-dans-eau comprend pour 100 parties en poids d'eau, de 300 à 50, de préférence de 200 à 120 parties de tensio-actif et de 300 à 50, de préférence de 180 à 100 parties de co-tensioactif.

5. Procédé de préparation d'une émulsion huile-dans-eau stable comprenant successivement :
a) le choix d'un tensio-actif de préférence d'un tensio-actif non-ionique, plus particulièrement choisi dans le groupe comprenant les esters d'acides gras de polyglycérol éventuellement éthoxylés, les éthoxylats d'alcools et d'un co-tensioactif choisi parmi les composés hydrophiles, de préférence comprenant au moins un groupe hydroxyle, choisis notamment dans la famille des polyols;
b) le mélange modéré, de préférence sans exercer de force de cisaillement, du tensio-actif, du co-tensioactif et d'une phase aqueuse dans des proportions données par la zone cristal-liquide ordonnée du diagramme de phase de ces constituants et/ou dans des proportions choisies de façon à ce que la structure du mélange de ces trois constituants observée au microscope en lumière polarisée présente des caractéristiques de biréfringence, le mélange étant réalisé jusqu'à l'obtention d'une pré-émulsion air-dans-eau blanche visible par observation au microscope en lumière polarisée, la surface des globules d'air présentant des caractéristiques de biréfringence ,
c) l'incorporation dans cette pré-émulsion air-dans-eau d'un corps gras simple ou complexe par mélange modéré, de préférence sans exercer de force de cisaillement,jusqu'à l'obtention d'un gel,
d) éventuellement l'addition d'une phase huileuse, et
e) éventuellement l'addition d'une phase aqueuse, de façon à obtenir une émulsion blanche.

6. Procédé selon la revendication 5, **caractérisé par le fait que** le corps gras simple ou complexe est ajouté par fractions successives sous agitation lente sans exercer de force de cisaillement.

7. Procédé selon la revendication 5 ou 6, comprenant une étape ultérieure f) de dilution à l'aide d'une phase aqueuse contenant éventuellement une substance active hydrophile par mélange modéré, de préférence sans exercer de force de cisaillement.

8. Procédé selon l'une quelconque des revendications 5 à 7, comprenant une étape supplémentaire g) d'addition par mélange modéré, de préférence sans exercer de force de cisaillement, d'une émulsion huile-dans-eau stable telle que définie à l'une quelconque des revendications 1 à 4 ou telle que préparée selon l'une quelconque des revendications 5 à 7, ou d'une émulsion huile-dans-eau classique.

9. Procédé de formulation d'une émulsion huile-dans-eau complexe comprenant les étapes selon lesquelles :
- on détermine les propriétés et fonctionnalités de l'émulsion huile-dans-eau finale souhaitée ;
- on choisit des émulsions élémentaires présentant chacune au moins l'une des propriétés ou fonctionnalités devant être présentées par l'émulsion finale;
- éventuellement on dilue au moins l'une des émulsions élémentaires avec une phase aqueuse;
- on mélange les différentes émulsions élémentaires, dont éventuellement certaines ont préalablement été diluées ;
- on dilue éventuellement avec une phase aqueuse ;
les étapes de dilution et de mélange étant réalisées de préférence sans exercer de force de cisaillement ; chacune desdites émulsions élémentaires étant une émulsion huile-dans-eau stable selon l'une quelconque des revendications 1 à 4 ou préparée selon l'une des revendications 5 à 8.

10. Emulsion selon l'une quelconque des revendications 1 à 4, ou préparée selon le procédé des revendications 5 à 9,
**caractérisée par le fait que** le diamètre moyen des particules ou gouttelettes d'huile est inférieur à 10µm environ, de préférence inférieur à 1µm environ, et plus préférentiellement encore compris entre 150 et 750nm environ.

11. Emulsion selon l'une quelconque des revendications 1 à 4, ou préparée selon le procédé des revendications. 5 à 9,
**caractérisée par le fait qu'**elle comprend au moins un composé actif lipophile et/ou au moins un composé actif hydrophile.

12. Emulsion selon l'une quelconque des revendications 1 à 4, ou préparée selon le procédé des revendications 5 à 9,
**caractérisée par le fait que** la teneur en tensioactif est au plus d'environ 20% en poids, de préférence d'au plus environ 10% en poids et plus préférentiellement encore d'au plus environ 5% en poids.

13. Emulsion selon l'une quelconque des revendications 1 à 4, ou préparée selon le procédé des revendications 5 à 9,
**caractérisée par le fait que** la teneur en huile est comprise entre 0,05% à 95% en poids d'huile, de préférence de 30 à 92% en poids d'huile.

14. Emulsion selon l'une quelconque des revendications 1 à 4, ou préparée selon le procédé des revendications 5 à 9,
**caractérisée par le fait que** la quantité d'agents actifs hydrosolubles, présents dans la phase aqueuse, est au plus d'environ 80% en poids du poids total de l'émulsion, plus généralement elle est d'au plus environ 10%, et de préférence de l'ordre.de 5% environ et la quantité d'agents actifs liposolubles, présents dans la phase huileuse, .est d'au plus 92% environ en poids, de préférence de 30 à 50% en poids et plus préférentiellement encore de 1 à 10% en poids du poids total de l'émulsion.

## Claims

1. A stable oil-in-water emulsion obtained from an air-in-water preemulsion comprising:
- a surfactant, preferably a nonionic surfactant, chosen more particularly from the group consisting of polyglycerol fatty acid esters which are optionally ethoxylated and of alcohol ethoxylates;
- a cosurfactant chosen from hydrophilic compounds, preferably comprising at least one hydroxyl group, which are chosen in particular from the family of the polyols;
- an aqueous phase;
in ratios chosen within the region of ordered liquid crystal structure of the phase diagram of these three components and/or in ratios chosen so that the structure of the mixture of these three constituents, observed under an optical microscope in polarized light, exhibits birefringence characteristics,
a simple or complex fatty substance being added to said preemulsion by moderate mixing, preferably without exerting a shear force, said fatty substance being chosen from the group comprising in particular fatty acid esters, waxes, butters, wax esters, natural, synthetic or mineral oils, hydrogenated oils and their mixtures.

2. The emulsion as claimed in claim 1, **characterized in that** the air-in-water preemulsion is a white paste, the constituent air globules of which can be observed in polarized light, their surfaces exhibiting birefringence characteristics.

3. The emulsion as claimed in claim 1 or claim 2, **characterized in that** the surfactant is chosen from the group of the glycerol derivatives consisting of lecithin derivatives, polyglycerol fatty acid esters which are optionally ethoxylated, preferably decaglycerol fatty acid esters with an HLB of greater than or equal to 13, even more preferably decaglyceryl laurate, myristate, stearate, isostearate or oleate, and their mixtures and **in that** the cosurfactant is a polyol, preferably diglycerol.

4. The emulsion as claimed in any one of claims 1 to 3, **characterized in that** the air-in-water preemulsion comprises, per 100 parts by weight of water, from 300 to 50, preferably from 200 to 120, parts of surfactant and from 300 to 50, preferably from 180 to 100, parts of cosurfactant.

5. A process for the preparation of a stable oil-in-water emulsion successively comprising:
a) the choice of a surfactant, preferably of a nonionic surfactant, chosen more particularly from the group consisting of polyglycerol fatty acid esters which are optionally ethoxylated and of alcohol ethoxylates, and of a cosurfactant chosen from hydrophilic compounds, preferably comprising at least one hydroxyl group, which are chosen in particular from the family of the polyols;
b) the moderate mixing, preferably without exerting a shear force, of the surfactant, of the cosurfactant and of an aqueous phase in proportions given by the ordered liquid crystal region of the phase diagram of these constituents and/or in proportions chosen so that the structure of the mixture of these three constituents observed under a microscope in polarized light exhibits birefringence characteristics, the mixing being carried out until a white air-in-water preemulsion is obtained which is visible by observation under a microscope in polarized light, the surface of the air globules exhibiting birefringence characteristics;
c) the incorporation in this air-in-water preemulsion of a simple or complex fatty substance by moderate mixing, preferably without exerting a shear force, until a gel is obtained;
d) optionally the addition of an oily phase; and
e) optionally the addition of an aqueous phase, so as to obtain a white emulsion.

6. The process as claimed in claim 5, **characterized in that** the simple or complex fatty substance is added in successive fractions with slow stirring without exerting a shear force.

7. The process as claimed in claim 5 or 6, comprising a subsequent stage f) of dilution using an aqueous phase optionally comprising a hydrophilic active substance by moderate mixing, preferably without exerting a shear force.

8. The process as claimed in any one of claims 5 to 7, comprising an additional stage g) of addition by moderate mixing, preferably without exerting a shear force, of a stable oil-in-water emulsion as defined in any one of claims 1 to 4 or as prepared according to any one of claims 5 to 7 or of a conventional oil-in-water emulsion.

9. A process for the formulation of a complex oil-in-water emulsion comprising the stages according to which:
- the properties and functions of the final oil-in-water emulsion desired are determined;
- individual emulsions each exhibiting at least one of the properties or functions which have to be exhibited by the final emulsion are chosen;
- optionally, at least one of the individual emulsions is diluted with an aqueous phase;
- the various individual emulsions, some of which have optionally been diluted beforehand, are mixed;
- the mixture is optionally diluted with an aqueous phase;
the dilution and mixing stages preferably being carried out without exerting a shear force;
each of said individual emulsions being a stable oil-in-water emulsion as claimed in any one of claims 1 to 4 or prepared according to one of claims 5 to 8.

10. The emulsion as claimed in any one of claims 1 to 4 or prepared according to the process of claims 5 to 9, **characterized in that** the mean diameter of the oil particles or droplets is less than 10 µm approximately, preferably less than 1 µm approximately and more preferably still between 150 and 750 nm approximately.

11. The emulsion as claimed in any one of claims 1 to 4 or prepared according to the process of claims 5 to 9, **characterized in that** it comprises at least one lipophilic active compound and/or at least one hydrophilic active compound.

12. The emulsion as claimed in any one of claims 1 to 4 or prepared according to the process of claims 5 to 9, **characterized in that** the content of surfactant is at most approximately 20% by weight, preferably at most approximately 10% by weight and more preferably still at most approximately 5% by weight.

13. The emulsion as claimed in any one of claims 1 to 4 or prepared according to the process of claims 5 to 9, **characterized in that** the oil content is between 0.05% and 95% by weight of oil, preferably from 30 to 92% by weight of oil.

14. The emulsion as claimed in any one of claims 1 to 4 or prepared according to the process of claims 5 to 9, **characterized in that** the amount of water-soluble active agents, which are present in the aqueous phase, is at most approximately 80% by weight of the total weight of the emulsion, more generally at most approximately 10% and preferably of the order of approximately 5%, and the amount of fat-soluble active agents, which are present in the oily phase, is at most approximately 92% by weight, preferably from 30 to 50% by weight and more preferably still from 1 to 10% by weight of the total weight of the emulsion.

## Patentansprüche

1. Stabile ÖI-in-Wasser-Emulsion, die aus einer Luft-in-Wasser-Voremulsion erhalten wird, umfassend:
- ein Tensid, vorzugsweise ein nichtionisches Tensid, das genauer aus der Gruppe, umfassend, gegebenenfalls ethoxylierte, Polyglycerin-Fettsäureester, Alkoholetoxylate ausgewählt ist;
- ein Cotensid, das aus den hydrophilen Verbindungen ausgewählt ist, die vorzugsweise mindestens eine Hydroxylgruppe umfassen, die insbesondere aus der Polyol-Familie ausgewählt sind,
- eine wässrige Phase,
in den Verhältnissen, die aus der Flüssigkristallstrukturzone ausgewählt sind, Ordinate des Phasendiagramms dieser drei Elemente, und/oder in den Verhältnissen, die derart ausgewählt sind, dass die Struktur der Mischung dieser drei Bestandteile, die unter dem optischen Mikroskop in polarisiertem Licht beobachtet werden, Doppelbrechungscharakteristiken aufweist,
wobei ein einfacher oder komplexer Fettstoff unter mäßigem Mischen, vorzugsweise ohne Ausübung von Scherkraft, zu der Voremulsion hinzugegeben wird, wobei der Fettstoff aus der Gruppe ausgewählt ist, die insbesondere Fettsäureester, Wachse, Butter, Wachsester, natürliche, synthetische oder mineralische Öle, gehärtete Öle und ihre Mischungen umfasst.

2. Emulsion nach Anspruch 1, **dadurch gekennzeichnet, dass** die Luft-in-Wasser-Emulsion eine weiße Paste ist, deren Luftkügelchen, die sie ausmachen, in polarisiertem Licht beobachtet werden können, wobei ihre Oberflächen Doppelbrechungscharakteristiken aufweisen.

3. Emulsion nach Anspruch 1 oder Anspruch 2, **dadurch gekennzeichnet, dass** das Tensid aus der Gruppe von Glycerinderivaten, umfassend Lecithinderivate, Polyglycerin-Fettsäureester, die gegebenenfalls ethoxyliert sind, vorzugsweise Decaglycerin-Fettsäureester mit einem HLB, der größer oder gleich 13 ist, noch bevorzugter Decaglycerin-Laurat, -Myristat, -Stearat, -Isostearat oder -Oleat und ihren Mischungen ausgewählt ist und **dadurch**, dass das Cotensid ein Polyol, vorzugsweise Diglycerin ist.

4. Emulsion nach einem der Ansprüche 1 bis 3, **dadurch gekennzeichnet, dass** die Luft-in-Wasser-Voremulsion für 100 Gewichtsteile Wasser 300 bis 50, vorzugsweise 200 bis 120 Teile an Tensid und 300 bis 50, vorzugsweise 180 bis 100 Teile an Cotensid umfasst.

5. Verfahren zur Herstellung einer stabilen Öl-in-Wasser-Emulsion, die nacheinander umfasst:
a) Auswählen eines Tensids, vorzugsweise eines nichtionischen Tensids, das genauer aus der Gruppe, umfassend Polyglycerin-Fettsäureester, die gegebenenfalls ethoxyliert sind, Alkoholethoxylate und ein Cotensid, das aus hydrophilen Verbindungen, die vorzugsweise mindestens eine Hydroxylgruppe umfassen, und zwar die aus der Polyol-Familie ausgewählt sind, ausgewählt ist;
b) mäßiges Mischen, vorzugsweise ohne Ausübung von Scherkraft, des Tensids, des Cotensids und einer wässrigen Phase in von der Flüssigkristallzone gegebenen Verhältnissen, welche die Ordinate des Phasendiagramms dieser Bestandteile ist, und/oder in Verhältnissen, die derart gewählt sind, dass die Struktur des Gemisches dieser drei Bestandteile, die unter dem Mikroskop in polarisiertem Licht beobachtet wird, Doppelbrechungscharakteristiken aufweist, wobei das Mischen durchgeführt wird, bis eine weiße Luft-in-Wasser-Voremulsion erhalten wird, die durch Beobachtung unter dem Mikroskop in polarisiertem Licht sichtbar ist, wobei die Oberfläche der Luftkügelchen Doppelbrechungscharakteristiken aufweist,
c) Unterziehen unter mäßigem Mischen eines einfachen oder komplexen Feststoffes unter diese Luft-in-Wasser-Voremulsion, vorzugsweise ohne Ausübung von Scherkraft, bis ein Gel erhalten wird,
d) gegebenenfalls Zugeben einer öligen Phase und
e) gegebenenfalls Zugeben einer wässrigen Phase, so dass eine weiße Emulsion erhalten wird.

6. Verfahren nach Anspruch 15, **dadurch gekennzeichnet, dass** der einfache oder komplexe Feststoff in aufeinander folgenden Fraktionen unter langsamem Rühren ohne Ausübung von Scherkraft hinzugegeben wird.

7. Verfahren nach Anspruch 5 oder 6, umfassend einen weiteren Schritt f) des Verdünnens mithilfe einer wässrigen Phase, die gegebenenfalls einen hydrophilen Wirkstoff enthält, durch mäßiges Mischen, vorzugsweise ohne Ausübung von Scherkraft.

8. Verfahren nach einem der Ansprüche 5 bis 7, umfassend einen zusätzlichen Schritt g) des Zugebens unter mäßigem Mischen, vorzugsweise ohne Ausübung von Scherkraft, einer stabilen Öl-in-Wasser-Emulsion, wie in einem der Ansprüche 1 bis 4 definiert oder wie nach einem der Ansprüche 5 bis 7 hergestellt, oder einer klassischen Öl-in-Wasser-Emulsion.

9. Verfahren zur Formulierung einer komplexen Öl-in-Wasser-Emulsion, umfassend die Schritte, nach denen:
- die Eigenschaften und Funktionalitäten der endgültigen gewünschten Öl-in-Wasser-Emulsion bestimmt werden;
- die elementaren Emulsionen ausgewählt werden, die jeweils mindestens eine der Eigenschaften oder Funktionalitäten aufweisen, welche die endgültige Emulsion aufweisen soll;
- gegebenenfalls mindestens eine der elementaren Emulsionen mit einer wässrigen Phase aufgelöst wird;
- die unterschiedlichen elementaren Emulsionen gemischt werden, von denen gegebenenfalls einige vorher verdünnt wurden;
- gegebenenfalls mit einer wässrigen Phase verdünnt wird;
wobei die Schritte des Verdünnens und des Mischens vorzugsweise ohne Ausübung von Scherkraft durchgeführt werden;
wobei jede der elementaren Emulsionen eine stabile ÖI-in-Wasser-Emulsion nach einem der Ansprüche 1 bis 4 ist oder nach einem der Ansprüche 5 bis 8 hergestellt ist.

10. Emulsion nach einem der Ansprüche 1 bis 4 oder hergestellt nach dem Verfahren der Ansprüche 5 bis 9, **dadurch gekennzeichnet, dass** der mittlere Durchmesser der Partikel oder Kügelchen aus Öl kleiner als ungefähr 10 µm ist, vorzugsweise kleiner als ungefähr 1 µm und noch bevorzugter zwischen ungefähr 150 und 750 nm liegt.

11. Emulsion nach einem der Ansprüche 1 bis 4 oder hergestellt nach dem Verfahren der Ansprüche 5 bis 9, **dadurch gekennzeichnet, dass** sie mindestens eine lipophile aktive Verbindung und/oder mindestens eine hydrophile aktive Verbindung umfasst.

12. Emulsion nach einem der Ansprüche 1 bis 4 oder hergestellt nach dem Verfahren der Ansprüche 5 bis 9, **dadurch gekennzeichnet, dass** der Gehalt an Tensiden höchstens ungefähr 20 Gew.-%, vorzugsweise höchstens ungefähr 10 Gew.-% und noch bevorzugter höchstens ungefähr 5 Gew.-% beträgt.

13. Emulsion nach einem der Ansprüche 1 bis 4 oder hergestellt nach dem Verfahren der Ansprüche 5 bis 9, **dadurch gekennzeichnet, dass** der Gehalt an Öl zwischen 0,05 Gew.-% und 95 Gew.-% Öl, vorzugsweise zwischen 30 und 92 Gew.-% Öl liegt.

14. Emulsion nach einem der Ansprüche 1 bis 4 oder hergestellt nach dem Verfahren der Ansprüche 5 bis 9, **dadurch gekennzeichnet, dass** die Menge an wasserlöslichen Wirkstoffen, die in der wässrigen Phase vorliegen, höchstens ungefähr 80 Gew.-% des Gesamtgewichtes der Emulsion beträgt, im Allgemeinen höchstens ungefähr 10 % beträgt und vorzugsweise im Bereich von ungefähr 5 % liegt und die Menge an fettlöslichen Wirkstoffen, die in der öligen Phase vorliegen, höchstens ungefähr 92 Gew.-%, vorzugsweise 30 bis 50 Gew.-% und noch bevorzugter 1 bis 10 Gew.-% des Gesamtgewichtes der Emulsion beträgt.
